# EUROPÄISCHE PATENTSCHRIFT

(11) **EP 1 053 215 B1**
(45) Veröffentlichungstag und Bekanntmachung des Hinweises auf die Patenterteilung: **22.05.2002**
(21) Anmeldenummer: 99910180.1
(22) Anmeldetag: 02.02.1999
(51) Int. Cl.: C07B 37/10, C07D 245/02

(54) **VERFAHREN ZUR DARSTELLUNG MAKROCYCLISCHER PRODUKTE DURCH RINGSCHLIESSENDE DIIN-METATHESE**
METHODS FOR PREPARING MACROCYCLIC PRODUCTS BY RING-CLOSING DIINE METATHESIS
PROCEDES DE PREPARATION DE PRODUITS MACROCYCLIQUES PAR METATHESE DIINE A FERMETURE DES COMPOSES CYCLIQUES

(30) Priorität: 06.02.1998 DE 19804673
(43) Veröffentlichungstag der Anmeldung: 22.11.2000
(73) Patentinhaber: Studiengesellschaft Kohle mbH, 45470 Mülheim an der Ruhr (DE)
(72) Erfinder: FÜRSTNER, Alois, D-45470 Mülheim an der Ruhr (DE); SEIDEL, Günter, D-45470 Mülheim an der Ruhr (DE); RUMBO, Antonio, D-45470 Mülhelm an der Ruhr (DE); MATHES, Christian, D-45470 Mülheim an der Ruhr (DE)
(74) Vertreter: von Kreisler, Alek, Dipl.-Chem.
(86) Internationale Anmeldenummer: EP9900674
(87) Internationale Veröffentlichungsnummer: WO9940047

(56) Entgegenhaltungen:
- A. FÜRSTNER: "Conformationally unbiased macrocyclization reactions by ring closing metathesis" JOURNAL OF ORGANIC CHEMISTRY, Bd. 61, Nr. 12, 1996, Seiten 3942-3943, XP002077144 EASTON US
- P. BERTINATO: "Studies toward a synthesis of epothilone A: stereocontrolled assembly of the acyl region and models for macrocyclization" JOURNAL OF ORGANIC CHEMISTRY, Bd. 61, Nr. 23, 1996, Seiten 8000-8001, XP002077143 EASTON US
- J. BOIVIN: "An efficient synthesis of large ring acetylenes" TETRAHEDRON LETTERS, Bd. 36, Nr. 32, 1995, Seiten 5737-5740, XP002103896 OXFORD GB
- A. FÜRSTNER: "Ring-closing metathesis of functionalized acetylene derivatives: a new entry into cycloalkynes" ANGEWANDTE CHEMIE INTERNATIONAL EDITION., Bd. 37, Nr. 12, 3. Juli 1998, Seiten 1734-1736, XP002103897 WEINHEIM DE

## Beschreibung

Die vorliegende Erfindung betrifft Verfahren zur Darstellung von makrocyclischen Produkten mit 9 oder mehr Ringatomen durch ringschließende Metathese von Diin-Substraten.

Unter Alkinmetathese versteht man die wechselseitige Umalkylidinierung von Alkinen gemäß Schema 1. 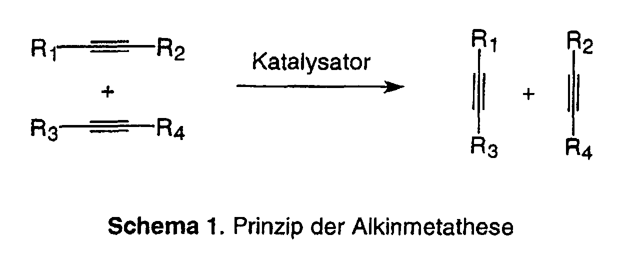 Reaktionen dieser Art werden in der Regel durch Metallverbindungen katalysiert (Übersichten: Schrock, R. R. *Polyhedron* **1995,** *14,* 3177; Ivin, K. J.; Mol, J. C. *Olefin Metathesis and Metathesis Polymerization*, Academic Press, New York, **1997,** S. 192-223). Im Gegensatz zur Metathese von Alkenen, die heute ein gut etabliertes Forschungsgebiet darstellt und zahlreiche Anwendungen auf die Darstellung technisch bedeutsamer Produkte gefunden hat (Übersichten: Ivin, K. J.; Mol, J. C. *Olefin Metathesis and Metathesis Polymerization,* Academic Press, New York, **1997;** Schuster, M. et al., *Angew. Chem*. **1997,** *109*, 2125), beschränkt sich die Anwendung der Alkinmetathese in der organischen Chemie auf die Darstellung spezieller Polymere (Weiss, K. et al., *Angew. Chem.* **1997,** *109,* 522), die ring-öffnende Polymerisation von Cycloalkinen (Krouse, S. A. et al., *Macromolecules* **1989,** *22*, 2569; Zhang, X-P. et al., *Macromolecules* **1994,** *27*, 4627), sowie auf die Dimerisierung bzw. die Kreuzmetathese acyclischer Alkine (Kaneta, N. et al., *Chem. Lett.* **1995,** 1055; Sancho, J. et al., *J. Mol. Cat.* **1982,** *15,* 75; Villemin, D. et al., *Tetrahedron Lett.* **1982,** 5139). Metathesen von Diinen führen zu polymeren Produkten durch acyclische Diinmetathese (Krouse, S. A. et al., *Macromolecules* **1989,** *22,* 2569) oder durch Cyclopolymerisation (Fox, H. H. et al. *J. Am. Chem. Soc*. **1994,** *116*, 2827; Koo, K.-M. et al., *Macromolecules* **1993,** *26*, 2485).

Als Katalysatoren oder Prä-Katalysatoren für Alkinmetathesen können sowohl heterogene als auch homogene Übergangsmetallverbindungen eingesetzt werden. Als katalytisch aktive Spezies werden Übergangsmetall-Alkylidin Komplexe bzw. Übergangsmetall-Carbin Komplexe angesehen (Katz, T. J. et al., *J. Am. Chem. Soc.* **1975,** *97*,1592), die entweder in isolierter Form den Reaktionsmischungen zugesetzt oder in situ aus geeigneten Prä-Katalysatoren gebildet werden können. Die katalytische Aktivität von Übergangsmetallverbindungen in Alkinmetathesen kann durch Zugabe geeigneter Additiva wie z.B. Phenolderivate (Mortreux, A. et al., *J. Chem. Soc. Chem. Commun.* **1974,** 786; Mortreux, A. et al., *J. Mol. Cat.* **1977,** *2*, 73; Villemin, D. et al., *Tetrahedron Lett.* **1982,** 5139), Aluminiumalkyle (Petit, M. et al., *J. Chem. Soc. Chem. Commun.* **1982,** 1385), oder SiO₂ (Mortreux, A. et al., Bull. *Soc. Chim. Fr.* **1972,** 1641; Mortreux, M. et al. *J. Mol. Cat.* **1980,** *8,* 97) erhöht werden.

Bevorzugte Katalysatoren oder Prä-Katalysatoren für Alkinmetathesen sind Mo(CO)₆ (Mortreux, A. et al., *J. Chem. Soc. Chem. Commun.* **1974,** 786; Mortreux, A. et al., *J. Mol. Cat.* **1977,** *2*, 73; Villemin, D. et al *Tetrahedron Lett.* **1982,** 5139; Tsonis, C. *React. Kinet. Catal. Lett.* **1992,** *46*, 359), MoO₂(acac)₂/Et₃Al (Petit, M. et al., *J. Chem. Soc. Chem. Commun.* **1982,** 1385), MoO₃/SiO₂ (Mortreux, A. et al., *Bull. Soc. Chim. Fr.* **1972,** 1641; Mortreux, M. et al. *J. Mol. Cat.* **1980,** *8,* 97), WO₃/SiO₂ (Pennella, F. et al., *Chem. Commun* **1968,** 1548), W(≡CCMe₃)(OR)₃ oder Mo(≡CCMe₃)(OR)₃ [R = CMe₃, CH(CF₃)₂, CMe₂CF₃, CMe(CF₃)₂, C(CF₃)₃, C₆H₃Me₂, C₆H₃i-Pr₂, C₆H₃t-Bu₂] (Übersicht: Schrock, R. R. *Polyhedron* **1995,** *14,* 3177; Sancho, J. et al., *J. Mol. Cat.* **1982,** *15,* 75; Weiss, K. in *Carbyne Complexes* [Fischer, H. et al., Eds.], Verlag Chemie, Weinheim, **1988,** S. 220), Re(≡CCMe₃)(=NAr)[OCMe(CF₃)₂]₂ (Schrock, R. R. et al., *J. Am. Chem. Soc.* **1988,** *110*, 2686; Weinstock, I. A. et al., *J. Am. Chem. Soc.* **1991,** *113*, 135), (Me₃CO)₃W≡W(OCMe₃) oder (Me₃CO)₃Mo≡Mo(OCMe₃) (Schrock, R. R. *Polyhedron* **1995,** *14*, 3177; Krouse, S. A. et al., *Macromolecules* **1989,** *22,* 2569; Zhang, X-P. et al., *Macromolecules* **1994,** *27,* 4627), und Komplexe die eine Re≡Re Dreifachbindung enthalten (Diefenbach S. P. US 4,698,451, 06. Okt. 1987; *Chem. Abstr.* **1988,** *108*, 40092m).

In der Literatur sind sowohl Diine als auch Cycloalkine bisher nur als Ausgangsmaterialien für Polymerisationsreaktionen via Alkinmetathese eingesetzt worden. Überraschenderweise fanden wir Jedoch, daß sich Diine. entsprechender Kettenlänge als Substrate in Gegenwart geeigneter Katalysatoren mit hoher Selektivität zu Cycloalkinen schließen lassen, sofern die gebildeten Cycloalkine 12 oder mehr Ringatome aufweisen (Schema 2).

Die Erfindung betrifft ein Verfahren zur Darstellung von carbo- oder heterocyclischen Produkten mit 12 oder mehr Ringatomen durch ringschließende Metathesereaktionen, dadurch gekennzeichnet, daß Diln-Substrate in Gegenwart von einem oder mehreren homogen oder heterogen im Reaktionsmedium vorliegenden Alkinmetathese-Katalysatoren umgesetzt werden.

Dieser gegenüber den bisher verwendeten Verfahren zur Darstellung von Cycloalkinen verbesserte und verkürzte Zugang zu dieser Substanzklasse ist von Bedeutung, da diverse Cycloalkine selbst als Antiblotika von Interesse sind (cf. Nicolaou K. C. *Angew. Chem*. **1991,** *103*, 1453), und sich durch bestehende Methoden In andere makrocyclische Produkte von wirtschaftlicher Relevanz wie z.B. Pharmaka, Pheromone, Agrochemikalien, Kronenether, Geruchsstoffe, Parfuminhaltsstoffe oder Geschmacksstoffe umsetzen lassen.

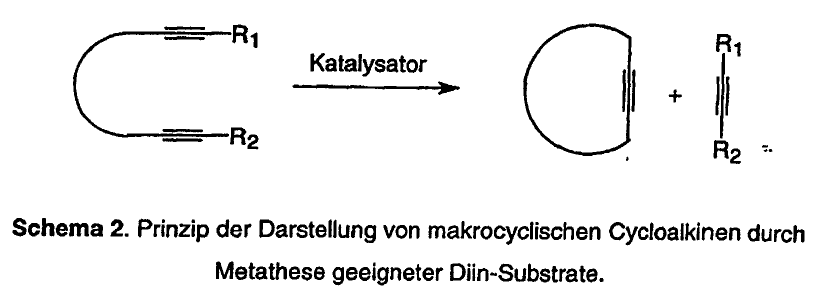

Die Selektivität dieser Reaktion hängt im einzelnen von der Struktur der Substrate, dem verwendeten Katalysator, den Reaktionsbedingungen, sowie der Ringspannung im erzeugten Cycloalkin ab. Die Bildung der Cycloalkine wird bei Durchführung der Reaktion bei hoher Verdünnung in einem organischem Lösungsmittel, das den Katalysator nicht desaktiviert, begünstigt. Bei der Wahl der Konzentration des Substrates im Reaktionsmedium ist dessen "effective molarity parameter" zu berücksichtigen (Mandolini, L. *Adv. Phys. Org. Chem.* **1986,** *22*,1). Bei höherer Konzentration können mit Hilfe der vorliegenden Erfindung durch Cyclodimerisierung der Diin-Substrate gemäß Schema 3 auch Cycloalkadiin Produkte erhalten werden.

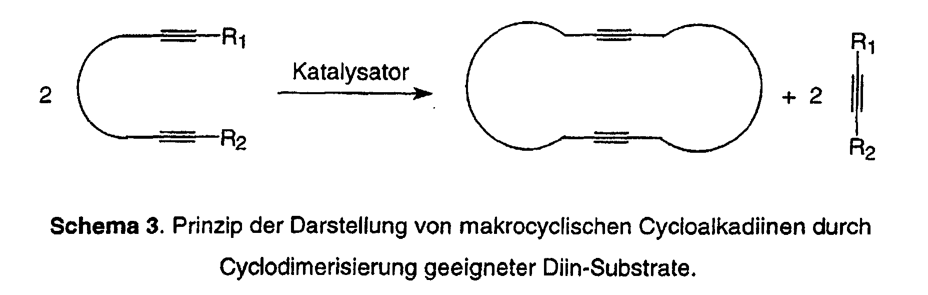

Als Katalysatoren oder Prä-Katalysatoren kommen in der vorliegenden Erfindung alle in Alkin-Metathesen aktiven Metallverbindungen in Betracht, unabhängig davon, ob sie im Reaktionsmedium homogen oder heterogen vorliegen. Die Katalysatoren können in isolierter Form eingesetzt oder in situ im Reaktionsmedium aus geeigneten Vorläufern erzeugt werden. Die eingesetzte Katalysatormenge ist nicht kritisch, wobei bevorzugte Katalysatormengen im Bereich von 0.01 - 10% bezogen auf das eingesetzte Substrat liegen.

Als bevorzugte Katalysatoren oder Prä-Katalysatoren dienen Übergangsmetall-Alkylidinkomplexe, Übergangsmetallverbindungen, die unter Reaktionsbedingungen Alkylidinkomplexe ausbilden, und Übergangsmetallverbindungen mit Metall≡Metall Dreifachbindungen.

Die im folgenden Text verwendeten Abkürzungen bedeuten: i-Pr = Isopropyl; t-Bu = tertiär Butyl; Ph = Phenyl; acac = Acetylacetonat; Ar = Aryl; gem = geminal; Me = Methyl.

Besonders bevorzugte Katalysatoren oder Prä-Katalysatoren sind Komplexe des allgemeinen Typs M(≡CR¹)(OR²)₃, mit
M = Mo, W
R¹ = C1-C20 Alkyl, Aryl, Alkenyl, Alkylthio, Dialkylamino, bevorzugt CMe₃, Ph
R² = C1-C20 Alkyl, Aryl, bevorzugt CMe₃, CH(CF₃)₂, CMe₂CF₃, CMe(CF₃)₂, C(CF₃)₃, C₆H₃Me₂, C₆H₃i-Pr₂, C₆H₃t-Bu₂

Besonders bevorzugte Katalysatoren oder Prä-Katalysatoren sind ebenfalls Komplexe des allgemeinen Typs Re(≡CR¹)(=NAr)(OR²)₂, mit
R¹ = C1-C20 Alkyl, Aryl, Alkenyl, bevorzugt CMe₃, Ph
Ar = C6-C20 Aryl
R² = C1-C20 Alkyl, Aryl, bevorzugt CMe₃, CH(CF₃)₂, CMe₂CF₃, CMe(CF₃)₂, C(CF₃)₃, C₆H₃Me₂, C₆H₃i-Pr₂, C₆H₃t-Bu₂

Besonders bevorzugte Katalysatoren oder Prä-Katalysatoren sind ebenfalls Komplexe des allgemeinen Typs (RO)₃M≡M(OR)₃, mit
M = Mo, W
R = C1-C20 Alkyl, bevorzugt CMe₃, CH(CF₃)₂, CMe₂CF₃, CMe(CF₃)₂, C(CF₃)₃

Bevorzugte Katalysatoren, die in situ im Reaktionsmedium erzeugt werden, entstehen aus Gemischen von Mo(CO)₆ und Phenolen. Besonders bevorzugte Katalysatoren entstehen bei Verwendung elektronenarmer Phenole wie Trifluormethylphenol, Bis(trifluormethyl)phenol, Fluorphenol, Difluorphenol, Pentafluorphenol, Chlorphenol, Dichlorphenol, Pentachlorphenol. Das Verhältnis Mo(CO)₆ : Phenol ist nicht kritisch; bevorzugte Verhältnisse Mo(CO)₆ : Phenol liegen im Bereich von 1:1 bis 1:1000, Bevorzugte Katalysatoren, die in situ im Reaktionsmedium erzeugt werden, entstehen ferner aus Gemischen von M[N(R¹)Ar]₃ und Halogenverbindungen vom Typ R²₂EX₂ oder R³₃SiX, wobei
M = Mo, W
R¹ = C1-C20 Alkyl, sekundär-Alkyl (sec-Alkyl), tertiär-Alkyt (t-Alkyl), Cycloalkyl, bevorzugt t-Bu
Ar= C6-C20 Aryl, bevorzugt C₆H₅, C₆H₄Me, C₆H₃Me₂, C₆H₃(i-Pr)₂, C₆H₃(t-Bu)₂, C₆H₂Me₃
R² = H, F, Cl, Br, I, C1-C20 Alkyl, Aryl
E=C, Si
R³ = C1-C20 Alkyl, Aryl, bevorzugt Methyl
X = F, Cl, Br, I
bevorzugt:
M=Mo
R¹ = t-Bu, i-Pr
Ar = C₆H₅, C₆H₄Me, C₆H₃Me₂, C₆H₃(iPr)₂, C₆H₃(t-Bu)₂, C₆H₂Me₃
R²=H,F,Cl,Br,I,C₆H₅
R³=Me, t-Bu, Ph, i-Pr.

Für die Darstellung von Verbindungen des Typs M[N(R¹)Ar]₃siehe: C. E. Laplaza et al., *J. Am. Chem. Soc*. **1996,** *118*, 8623.

Als Additive, die die Aktivität der verwendeten Katalysatoren erhöhen, werden Phenole, geminal-Dihalogenalkane (gem-Dihalogenalkane) oder Halogensilane eingesetzt, z.B. Phenol Trifluormethylphenol, Bis(trifluormethyl)phenol, Fluorphenol, Difluorphenol, Pentafluorphenol, Chlorphenol, Dichlorphenol, Pentachlorphenol, Dichlormethan, Dibrommethan, Diiodmethan, Chloroform, Bromoform, lodoform, Tetrachlorkohlenstoff, Tetrabromkohlenstoff, Tetraiodkohlenstoff, α,α-Dichlortoluol, Trimethylchlorsilan, Dimethyldichlorsilan, Trimethylbromsilan, Dimethyl(t-butyl)chlorsilan, Dimethylphenylchlorsilan.

Die in der vorliegenden Erfindung als Substrate verwendeten Diine können eine oder mehrere funktionelle Gruppen in Form von Substituenten an der Kette oder Heteroatomen innerhalb der Kette enthalten. Dies umfaßt unter anderem verzweigte oder unverzweigte Alkylreste, aromatische oder nicht aromatische carbocyclische Ringe, aromatische oder nicht aromatische Stickstoff-, Sauerstoff-, Schwefel- oder Phosphorhaltige heterocyclische Ringe, Carbonsäuren, Ester, Ether, Epoxide, Silylether, Thioether, Thioacetale, Disulfide, Alkohole, Anhydride, Imine, Silylether, Silylenolether, Ammoniumsalze, Amine, Amide, Nitrile, Perfluoralkyl-Gruppen, gem-Dialkylgruppen, Alkene, Halogene, Ketone, Ketale, Aldehyde, Acetale, Carbamate, Carbonate, Urethane, Harnstoffe, Sulfonate, Sulfone, Sulfonamide, Sulfoxide, Phosphate, Phosphonate, Nitro-Gruppen, Organosilan-Einheiten oder Metallzentren. Die Anwesenheit der genannten funktionellen Gruppen in den Substraten kann die Bildung der makrocyclischen Cycloalkinprodukte begünstigen. Repräsentative Beispiele sind in Tabelle 1 und in den Beispielen zusammengefaßt.

Die als Substrate verwendeten Diine können durch strukturelle Elemente wie z.B. chirale Zentren, Wasserstoffbrückenbindungen, supramolekulare Strukturen, rigide Rückgrade, Koordination an Metallzentren konformativ für den Ringschluß prä-organisiert sein. Es können auch Substrate verwendet werden, die keines dieser strukturellen Elemente aufweisen und daher konformativ flexibel sind. Die Substrate können in trägergebundener Form vorliegen.

Bevorzugte Substrate sind Diine mit R₁, R₂ ≠ H. Besonders bevorzuge Substrate sind solche Diine, bei denen die Reste R₁ und R₂ in Schemata 2 und 3 so gewählt werden, daß ein niedrigmolekulares und damit leicht flüchtiges Alkin R₁C≡CR₂ (z. B. 2-Butin, 2-Hexin, 3-Hexin) als Nebenprodukt der Bildung des makrocyclischen Cycloalkins entsteht.

Die Reaktionen werden so durchgeführt, daß die jeweiligen Substrate mit dem homogenen oder heterogenen Katalysator in Kontakt gebracht werden. In der Regel geschieht dies durch Vermischen einer Lösung oder Suspension des Substrates mit einer Lösung oder Suspension des Katalysators. Je nach verwendetem Katalysator und Substrat kann die Reaktionstemperatur schwanken, wobei bevorzugt bei -30°C bis +200°C gearbeitet wird. Die Reaktionszeit ist nicht kritisch und kann zwischen wenigen Minuten und einigen Tagen liegen. Die Reaktionen werden bevorzugt unter Schutzgasatmosphäre (z. B. Argon, Stickstoff, Helium) durchgeführt.

Im allgemeinen werden für ringschließende Diinmetathesen zu makrocyclischen Cycloalkinen Kohlenwasserstoffe (z. B. Hexan, Octan, Petrolether, Toluol, Xylole, Cumol, Decalin) oder halogenierte Kohlenwasserstoffe (z. B. Chlorbenzol, Brombenzol, Fluorbenzol, Trifluormethylbenzol, Dichlorbenzol, Trichlorbenzol, Tetrachlorkohlenstoff, 1,2-Dichlorethan) als Lösungsmittel bevorzugt. Bei Wahl geeigneter Katalysatoren sind auch andere Lösungsmittel wie z. B. Acetonitril, Tetrahydrofuran, 1,4-Dioxan, Dimethoxyethan, Dimethylformamid, Dimethylsulfoxid, Phenol einsetzbar. Auch Mischungen dieser Lösungsmittel können verwendet werden.

Die Reaktionen können bei Drücken kleiner als Atmosphärendruck durchgeführt werden. Das Anlegen von Unterdruck kann zum Entfernen flüchtiger Nebenprodukte R₁C≡CR₂ führen und so die erreichte Ausbeute an Cycloalkin verbessern. Der jeweils anwendbare Unterdruck richtet sich nach den spezifischen Eigenschaften des Substrats, des gebildeten Cycloalkins, des als Nebenprodukt gemäß Schemata 2 und 3 anfallenden Alkins R₁C≡CR₂, des verwendeten Lösungsmittels, sowie etwaiger Additiva. Auch durch das Durchleiten eines inerten Gasstromes durch die Reaktionslösung kann das niedermolekulare Nebenprodukt R₁C≡CR₂ aus dem Reaktionsgemisch ausgetrieben und so die Ausbeute an Cycloalkin verbessert werden.

Die Aufarbeitung der Reaktionsgemische und die Reinigung der Produkte ist nicht kritisch und richtet sich nach den jeweiligen physikalischen Eigenschaften der erzeugten Produkte und/oder der unumgesetzten Substrate. Bevorzugte Aufarbeitungs- und Reinigungsmethoden sind Destillation, Sublimation, Kristallisation, Chromatographie, Filtration, und Extraktion.

Die gemäß der vorliegenden Erfindung zugänglichen makrocyclischen Cycloalkine können für die Synthese zahlreicher Folgeprodukte genutzt werden, z. B. durch Reduktion, Oxidation, oder Cycloaddition der Dreifachbindung, sowie durch Additionen an die Dreifachbindung. Von besonderer Bedeutung ist die Möglichkeit, daß die durch die vorliegende Erfindung zugänglichen makrocyclischen Cycloalkine durch geeignete Reaktionen (z.B. Halbhydrierung, Hydrometallierung, Carbometallierung) zu makrocyclischen Cycloalkenen mit einheitlicher Konfiguration der Doppelbindung umgesetzt werden können.

Makrocyclische Cycloalkene mit einheitlicher Konfiguration der Doppelbindung sind in der Regel durch Ringschluß-Metathese von Dienen (RCM) nicht direkt zugänglich. RCM liefert meist Gemische der jeweiligen (E)- und (Z)-lsomere, wobei häufig das (E)-lsomere bevorzugt entsteht (Schuster M. al. *Angew. Chem.* **1997,** *109,* 2125; Fürstner, A. *Topics in Catalysis* **1997,** *4*, 285; Fürstner, A. et al. *Synthesis* **1997,** 792). Die vorliegende Erfindung ermöglicht es jedoch, makrocyclische, (Z)-konfigurierte Cycloalkene selektiv darzustellen, indem die durch ringschließende Metathese von Diinen erhaltenen Cycloalkine mit Hilfe geeigneter Reaktionen wie z. B. Halbhydrierung oder Hydrometallierung/Protonierung (Übersichten: March, J. *Advanced Organic Chemistry*, 4^{th} Ed., Wiley, New York, **1992,** S. 771ff; Marvell, E. N. et al. *Synthesis* **1973,** 457; Fürstner, A. et al. *J. Org. Chem.* **1997,** *62,* 2332) umgesetzt werden. Dabei können die durch die vorliegende Erfindung zugänglichen Cycloalkine zunächst isoliert und im Anschluß nach einer geeigneten Methode in das (Z)-konfigurierte Cycloalken überführt werden. Alternativ können die Bildung des makrocyclischen Cycloalkins durch ringschließende Alkinmetathese eines Diin-Substrats sowie dessen Überführung in ein makrocyclisches, (Z)-konfiguriertes Cycloalken in einem einzigen Reaktionsansatz im Sinn eines integrierten chemischen Verfahrens sukzessive durchgeführt werden.

Makrocyclische Cycloalkene mit (Z)-konfigurierter Doppelbindung werden vielfach als Antibiotika, Pharmaka für die Human- oder Veterinärmedizin, Pheromone, Geruchsstoffe, Parfuminhaltsstoffe etc. eingesetzt. Ein repräsentatives Beispiel für die Synthese eines pharmazeutisch relevanten makrocyclischen Produkts durch Oxidation eines makrocyclischen Cycloalkens sind Epothilon bzw. Analoga dieser Verbindung. Wird das zur Synthese von Epothilon bzw. dessen Analoga benötigte makrocyclische, (Z)-konfigurierte Cycloalken durch RCM dargestellt, so fallen in der Regel (E)/(Z)-Gemische an, von denen jedoch nur das jeweilige (Z)-Alken durch Epoxidierung der Doppelbindung in Epothilon bzw. in Analoga dieses Naturstoffs mit korrekter Konfiguration der stereogenen Zentren des gebildeten Epoxids überführt werden kann (Nicolaou, K. C. et al. *Angew. Chem.* **1996,** *108,* 2554; Meng, D. *J. Am. Chem. Soc.* **1997,** *119,* 2733; Taylor, R. E. *Tetrahedron Lett.* **1997,** 2061; Schinzer, D. et al. *Angew. Chem.* **1997,** *109,* 543; Yang, Z. *Angew. Chem.* **1997,** *109,* 170; Bertinato, P. *J. Org. Chem.* **1996,** *61,* 8000; Nicolaou, K. C. et al. *J. Am. Chem. Soc.* **1997,** *119,* 7960; Nicolaou K. C. et al. *Nature* **1997,** *387*, 268; Nicolaou, K. C. et al. *Chem. Eur. J.* **1997,** *3*, 1957; Nicolaou K. C. et al., *Angew. Chem.* **1997,** *109*, 2181). Diese und verwandte Synthesen lassen sich mit Hilfe der vorliegenden Erfindung durch Bildung des makrocyclischen Cycloalkins und dessen anschließende Halbreduktion zum (Z)-Cycloalken stereoselektiv gestalten und damit erheblich verbessern.

Die im folgenden angeführten Beispiele beschreiben prototypische Ringschlußreaktionen von Diinen zu makrocyclischen Produkten mit Hilfe von Alkin-Metathese Katalysatoren unter bevorzugten Bedingungen, sollen jedoch in keiner Weise den Umfang, die Anwendungsbreite oder die Vorteile der vorliegenden Erfindung einschränken.

### BEISPIEL 1

### Cyclisierung von Hexandicarbonsäure bis(3-pentinyl)ester

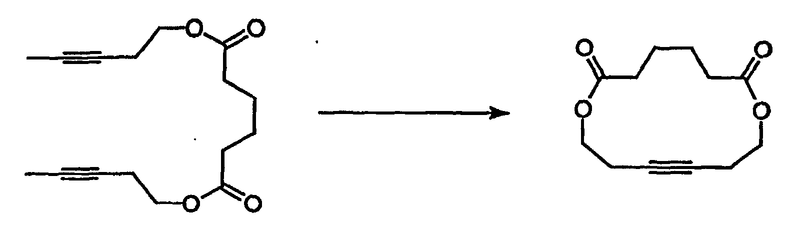

In einer Apparatur bestehend aus einem Zweihalskolben mit aufgesetzter Destillationsbrücke und einer auf -78°C gekühlten Vorlage wird eine Lösung von Hexandicarbonsäure bis(3-pentinyl)ester (155 mg, 0.56 mmol) in 1,2,4-Trichlorbenzol (30 mL) mit W(≡CCMe₃)(OCMe₃)₃ (8 mg) versetzt. Man evakuiert die Apparatur auf 2000 Pa (20 mbar) und erhitzt das Reaktionsgemisch auf 80°C. Nach 4h wird weiteres W(≡CCMe₃)(OCMe₃)₃ (8 mg) nachgegeben und die Lösung anschließend 13h bei 80°C 2000 Pa (20 mbar) gerührt. Abdestillieren des Lösungsmittels im Hochvakuum und säulenchromatographische Reinigung des Rückstandes (Eluationsmittel Hexan/Ethylacetat 4:1) ergibt das Cycloalkin als farblose Kristalle (100 mg, 79%). Mp = 106-107°C. ¹H-NMR: δ = 4.14 (t, 4H, J = 5.5), 2.53 (t, 4H, J = 5.6 ), 2.40 (m, 4H), 1.76 (m, 4H). ¹³C-NMR: δ = 173.0, 77.8, 62.4, 34.8, 24.9, 19.0. MS, m/z (rel Intensität): 224 (< 1), [M⁺], 179 ( < 1), 166 (1), 152 (1), 137 (1), 129 (3), 111 (7), 101 (4), 78 (100), 66 (21), 55 (10), 41 (8). C₁₂H₁₆O₄ (224.3) ber.: C 64.24. H 7.18; gefunden: C 64.14. H 7.15.

### BEISPIEL 2

### Cyclisierung von Hexandicarbonsäure bis(3-pentinyl)ester

Eine Lösung von Hexandicarbonsäure bis(3-pentinyl)ester (105 mg) und W(≡CCMe₃)(OCMe₃)₃(11 mg) in Toluol (20 mL) wird 1h bei 80°C unter Ar gerührt. Man destilliert das Lösungsmittel im Vakuum ab, reinigt den verbleibenden Rückstand säulenchromatographisch (Hexan/Ethylacetat 4/1), und erhält das gewünschte Cycloalkin als farblose Kristalle (59 mg, 69 %). Analytische Daten wie unter Beispiel 1 angeführt.

### BEISPIEL 3

### Cyclisierung von Hexandicarbonsäure bis(3-pentinyl)ester

Eine Lösung von Hexandicarbonsäure bis(3-pentinyl)ester (121 mg) und W(≡CCMe₃)(OCMe₃)₃ (12 mg) in Chlorbenzol (20 mL) wird 2 h bei 80°C unter Ar gerührt. Man destilliert das Lösungsmittel im Vakuum ab, reinigt den verbleibenden Rückstand säulenchromatographisch (Hexan/Ethylacetat 4/1), und erhält das gewünschte Cycloalkin als farblose Kristalle (70 mg, 73 %). Analytische Daten wie unter Beispiel 1 angeführt.

### BEISPIEL 4

### Cyclisierung und Cyclodimerislerung von 10-Dodecln-1-yl 10-dodecinoat

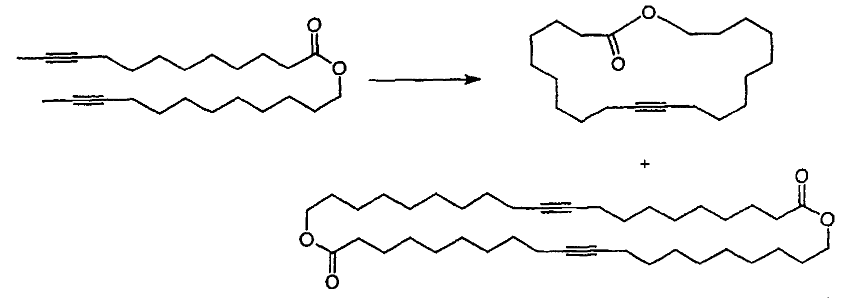

Eine Lösung von 10-Dodecin-1-yl 10-dodecinoat (139 mg, 0.39 mmol) und W(≡CCMe₃)(OCMe₃)₃ (9 mg) in Chlorbenzol (50 mL) wird 10h auf 80°C erhitzt. Nach Abdestillieren des Lösungsmittels und Säulenchromatographie des Rückstandes (Hexan/Ethylacetat 20/1) erhält man das Cycloalkin als farblose Kristalle (62 mg, 52%), sowie das Cycloalkadiin (Cyclodimerisierungsprodukt) als farbloses, kristallines Produkt.
Daten des Cycloalkins: ¹H-NMR: δ = 4.12 (t, 2H, J = 5.8), 2.32 (t, 2H, J = 6.8), 2.16 (m, 4H), 1.64 (m, 4H), [1.46 (m), 1.32 (m); 18H].- ¹³C-NMR: δ = 173.8, 80.7, 80.5, 63.9, 34.6, 29.39,29.36, 29.2, 28.8 (2C), 28.6 (2C), 28.4 (2C), 28.3, 28.1, 25.9, 25.3, 18.5, 18.4.- MS, m/z (rel. Intensität): 306 (35) [M⁺], 277 (4), 264 (5), 250 (3), 209 (6), 192 (19), 178 (34), 164 (40), 149 (24), 135 (54), 121 (61), 107 (43), 95 (71), 81 (97), 67 (98), 55 (100), 41 (82), 29 (25). C₂₀H₃₄O₂ (306.5) ber.: C 78.37. H 11.19; gefunden: C 77.55. H 11.07.
Daten des Cycloalkadiins: ¹H-NMR: δ = 4.07 (t, 4H, J = 6.6), 2.29 (t, 4H, J = 7.4), 2.14 (m, 8H), 1.62 (m, 8H), 1.47 (m, 8H), 1.4-1.2 (36H).- ¹³C-NMR: δ = 173.9, 80.27, 80.25, 64.3, 34.4, 29.3, 29.1, 29.06, 28.98, 28.96, 28.88, 28.62, 28.57, 25.9, 25.0, 18.7.- MS, m/z (rel. Intensität): 612 (99) [M⁺], 584 (7), 557 (5), 515 (6), 469 (5), 401 (8), 387 (11), 373 (13), 359 (18), 345 (19), 147 (16), 135 (26), 121 (32), 107 (33), 95 (65), 81 (88), 67 (87), 55 (100).

### BEISPIEL 5

### Cyclisierung von N,N-Bis(10-dodecinoyl)ethan-1,2-diamin

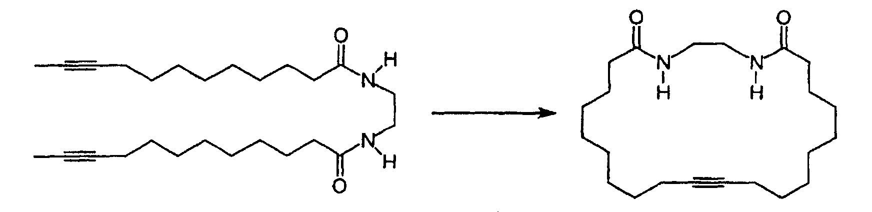

Eine Suspension von N,N-Bis(10-dodecinoyl)ethan-1,2-diamin (142 mg, 0.34 mmol) und W(≡CCMe₃)(OCMe₃)₃ (8 mg) in Chlorbenzol (20 mL) wird 3h bei 80°C gerührt. Durch Abdestillieren des Lösungsmittels erhält man das Cycloalkin. Eine analytisch reine Probe wird durch Extraktion des Katalysators aus dem Produkt erhalten. MS m/z (rel. Intensität): 362 (82) [M⁺], 334 (17), 319 (9), 303 (8), 279 (7), 265 (7), 249 (9), 237 (16), 221 (22), 206 (14), 178 (13), 168 (13), 154 (10), 135 (14), 126 (10), 95 (40), 81 (51), 67 (67), 55 (91), 44 (79), 41 (73), 30 (100).

### BEISPIEL 6

### Cyclisierung von Hexandicarbonsäure bis(3-pentinyl)ester in THF

Eine Lösung von Hexandicarbonsäure bis(3-pentinyl)ester (89 mg) und W(≡CCMe₃)(OCMe₃)₃ (18 mg) in Tetrahydrofuran (15 mL) wird 22 h unter Ar zum Rückfluß erhitzt. Man destilliert das Lösungsmittel im Vakuum ab, reinigt den verbleibenden Rückstand säulenchromatographisch (Hexan/Ethylacetat 4/1), und erhält das gewünschte Cycloalkin als farblose Kristalle (46 mg, 64 %). Analytische Daten wie unter Beispiel 1 angeführt.

### BEISPIEL 7

### Cyclisierung von Hexandicarbonsäure bis(3-pentinyl)ester mit W(≡CPh)(OCMe₃)₃

Eine Lösung von Hexandicarbonsäure bis(3-pentinyl)ester (271 mg) und W(≡CPh)(OCMe₃)₃(25 mg) in Toluol (30 mL) wird 1 h unter Ar auf 80°C erwärmt. Man destilliert das Lösungsmittel im Vakuum ab, reinigt den verbleibenden Rückstand säulenchromatographisch (Hexan/Ethylacetat 4/1), und erhält das gewünschte Cycloalkin als farblose Kristalle (134 mg, 61 %). Analytische Daten wie unter Beispiel 1 angeführt.

### BEISPIEL 8

### Cyclisierung mit Hilfe von Mo(CO)₆/p-Chlorphenol als Alkinmetathese-Katalysator

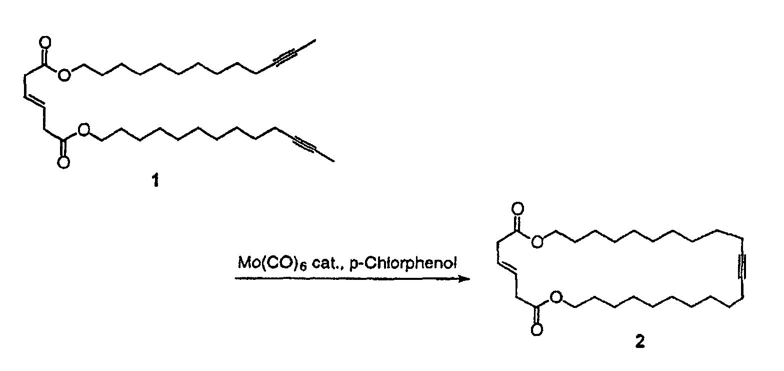

Eine Lösung des Diin Substrats **1** (200 mg), Mo(CO)₆ (5 mg) und p-Chlorphenol (55 mg) in Chlorbenzol (75 mL) wird 3 h auf 120°C erhitzt. Während der Reaktion wird ein leichter Argon-Strom durch das Reaktionsgemisch durchgeleitet. Zur Aufarbeitung werden flüchtige Bestandteile im Vakuum abkondensiert und der verbleibende Rückstand säulenchromatographisch gereinigt (Eluationsmittel Hexan/t-Butylmethylether 20/1). Man erhält das 30-gliedrige Cycloalkin **2** als farblosen Feststoff (126 mg, 70%). ¹H-NMR: δ = 5.63 (dt, 2H), 4.01 (t, 4H), 3.00 (dd, 4H, J = 4, 1.6), 2.08 (t, 4H, J = 6.5), 1.36 (m, 8H), 1.23 (m, 18H). ¹³C-NMR: δ = 171.8, 126.3, 80.8, 65.2, 38.6, 29.7, 29.6, 29.5, 29.4, 29.1, 28.9, 28.8, 26.2, 19.0.

### BEISPIEL 9

### Darstellung von Ambrettolid durch Diin-Metathese und anschließende Halbhydrierung

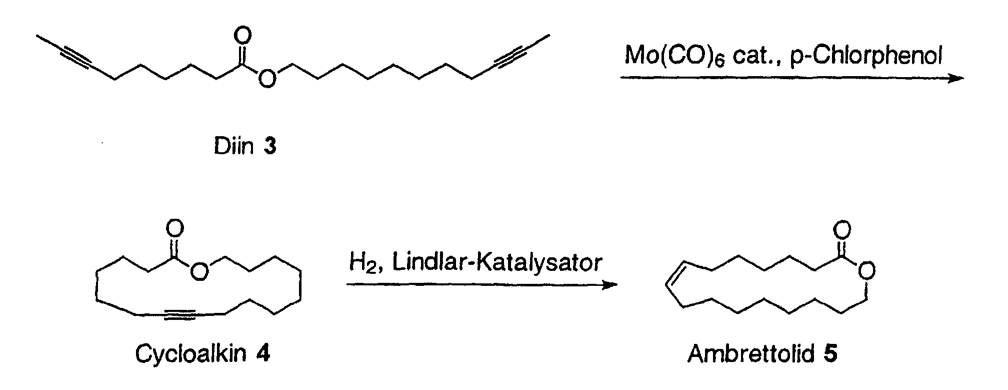

Eine Lösung des Diins **3** (1.0 g), Mo(CO)₆ (42 mg) und p-Chlorphenol (425 mg) in Chlorbenzol (150 mL) wird 19 h auf 120°C erhitzt. Während der Reaktionszeit wird ein leichter Argonstrom über eine Gaseinleitung durch das Reaktionsgemisch geleitet. Zur Aufarbeitung werden flüchtige Bestandteile im Vakuum abkondensiert und der verbleibende Rückstand säulenchromatographisch gereinigt (Eluationsmittel Hexan/t-Butylmethylether 20/1). Man erhält das Cycloalkin **4** als farblosen Syrup (568 mg, 69%), welcher die folgenden analytischen Daten aufweist: ¹H-NMR: δ = 4.10 (t, 2H, J = 5.2), 2.28 (t, 2H, J = 7), 2.13 (m, 4H), 1.60 (m, 4H), 1.15 (m, 14H). ¹³C-NMR: δ = 174.4, 80.7, 80.6, 64.3, 35.0, 28.8, 28.7, 28.5, 28.46, 28.40, 27.4, 25.3, 19.1, 18.9.
Ein Reaktionsgemisch bestehend aus Cycloalkin **4** (154 mg), Chinolin (60 µL) und Lindlar Katalysator (= 5% Pd auf Calciumcarbonat, vergiftet mit Blei) (60 mg) in Hexan (3 mL) wird 1.5 h unter einer Atmosphäre von Wasserstoff (1 atm) gerührt. Man filtriert den Katalysator ab, wäscht das Filtrat mit wässiger HCl (5%), trocknet die organische Phase über Na₂SO₄ und entfernt das Lösungsmittel im Vakuum. Anschließende Säulenchromatographie (Eluationsmittel Hexan/t-Butylmethylether 20/1) ergibt Ambrettolid **5** (138 mg, 92%). ¹³C-NMR: δ = 174.3, 130.5, 130.4, 64.1, 34.9, 29.8, 29.1, 29.0, 28.9, 28.8, 28.7, 28.0, 27.3, 27.1, 25.7, 25.6.

### BEISPIEL 10

### Cyclisierung von Hexandicarbonsäure bis(3-pentinyl)ester mit Hilfe von Mo[N(t-Bu)(3,5-C₆H₃Me₂)]₃/CH₂Cl₂ als Diin-Metathesekatalysator

Ein Reaktionsgemisch bestehend aus Hexandicarbonsäure bis(3-pentinyl)ester (60 mg) und Mo[N(t-Bu)(3,5-C₆H₃Me₂)]₃ (13.5 mg) (dargestellt gemäß der Literatur: C. E. Laplaza et al. *J. Am. Chem. Soc.* **1996,** *118*, 8623) in Toluol (15 mL) und CH₂Cl₂ (50 µL) wird 19 h unter Argon auf Rückfluß erhitzt. Man destilliert das Lösungsmittel im Vakuum ab, reinigt den verbleibenden Rückstand säulenchromatographisch (Hexan/Ethylacetat 4/1), und erhält das gewünschte Cycloalkin als farblose Kristalle (38.8 mg, 80 %). Analytische Daten wie unter Beispiel 1 angeführt.
Anstelle von CH₂Cl₂ können zur Aktivierung der Molybdänkomponente auch CHCl₃, CCl₄, CH₂Br₂, CH₂I₂, α,α-Dichlortoluol oder Trimethylchlorsilan verwendet werden. Die Reaktion kann analog in Dichlormethan als Lösungsmittel durchgeführt werden.

### BEISPIEL 11

### Diin-Metathese mit Hilfe von Mo[N(t-Bu)(3,5-C₆H₃Me₂)]₃/CH₂Cl₂

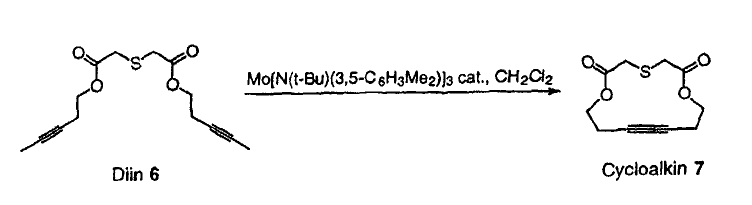

Ein Reaktionsgemisch bestehend aus Diin **6** (91.5 mg) und Mo[N(t-Bu)(3,5-C₆H₃Me₂)]₃(20.2 mg) (dargestellt gemäß der Literatur: C. E. Laplaza et al. *J. Am. Chem. Soc.* **1996,** *118,* 8623) in Toluol (15 mL) und CH₂Cl₂ (50 µL) wird 22 h unter Argon auf 80°C erhitzt. Man destilliert das Lösungsmittel im Vakuum ab, reinigt den verbleibenden Rückstand säulenchromatographisch (Hexan/Ethylacetat 8/1) und erhält das gewünschte Cycloalkin **7** als farblosen Syrup (62 mg, 84 %). Analytische Daten: ¹H-NMR: δ = 4.28 (t, 4H, J = 5.4), 3.43 (s, 4H), 2.49 (t, 4H). ¹³C-NMR: δ = 169.4, 78.1, 62.9, 34.6 (2x), 19.6. MS: *m*/*z* (rel. Intensität): 228 (56, [M⁺]), 78 (100).

### BEISPIEL 12

### Diin-Metathese mit Hilfe von Mo[N(t-Bu)(3,5-C₆H₃Me₂)]₃/CH₂Cl₂

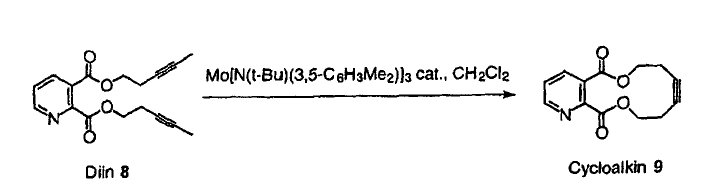

Ein Reaktionsgemisch bestehend aus Diin **8** (511 mg) und Mo[N(t-Bu)(3,5-C₆H₃Me₂)]₃(104.4 mg) (dargestellt gemäß der Literatur: C. E. Laplaza et al. *J. Am. Chem. Soc.* **1996,** *118*, 8623) in Toluol (82 mL) und CH₂Cl₂ (160 µL) wird 20 h unter Argon auf 80°C erhitzt. Man destilliert das Lösungsmittel im Vakuum ab, reinigt den verbleibenden Rückstand säulenchromatographisch (Hexan/Ethylacetat 4/1) und erhält das gewünschte Cycloalkin **9** als farblosen Syrup (369 mg, 88 %). Analytische Daten: ¹H-NMR: δ = 8.76 (dd, 1H, J = 4.9, 1.9), 8.12 (dd, 1H, J = 7.9, 1.5), 7.52 (dd, 1H, J = 4.9), 4.63 (t, 2H, J = 5.5), 4.42 (t, 2H, J = 5.6), 2.57 (m, 4H). ¹³C-NMR: δ = 166.1, 165.8, 151.1, 151.0, 137.1, 128.7, 125.1, 79.2, 78.8, 63.4, 63.1, 20.0, 19.4). MS: *m*/*z* (rel. Intensität): 245 (2, [M⁺]), 78 (100).

### BEISPIEL 13

### Darstellung eines 11-gliedrigen Cycloalkins: Cyclisierung und Cyclodimerisierung von Dimethylmalonsäure bis(3-pentinyl)ester

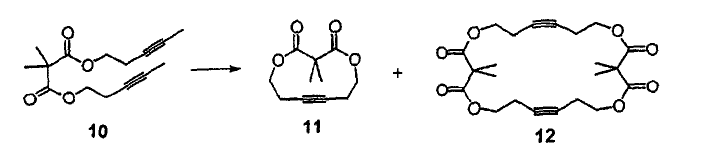

Eine Lösung von Dimethylmalonsäure bis(3-pentinyl)ester **10** (262 mg) und W(≡CCMe₃)(OCMe₃)₃ (28 mg) in Chlorbenzol (60 mL) rührt man 1 h bei 80°C unter Argon. Nach Abdestillieren des Lösungsmittels bei 12 mbar und säulenchromatographischer Reinigung des Rückstandes isoliert man wachsartig kristallines Cycloalkin **11** (97 mg, 47 %) sowie kristallines Cycloalkadiin **12** (87 mg, 42 %) und gewinnt wenig Diin **10** zurück (37 mg, 14 %).
Daten des Cycloalkins **11**: ¹H-NMR: δ = 4.29 (t, 4H, J = 5.8), 2.43 (t, 4H), 1.43 (s, 6H).- ¹³C-NMR: δ= 172.0, 79.8, 61.8, 50.0, 22.1, 19.7, - MS, *m*/*z* (rel. Intensität): 152 (2) [M-58], 137 (3), 111 (5), 87 (5), 78 (100), 70 (49), 66 (17), 65 (18), 41 (16).-C₁₁H₁₄O₄(210.2) ber.:C 62.85. H 6.71; gef.: C 63.01. H 6 67.
Daten des Cycloalkadiins **12**: ¹H-NMR: δ = 4.15 (t, 8H, J = 6.8), 2.53 (t, 8H), 1.43 (s, 12H). - ¹³C-NMR: δ = 172.4, 77.3, 63.5, 49.4, 22.5, 18.8.- MS, *m*/*z* (rel. Intensität): 420 (5) [M⁺], 174 (8), 156 (48), 141 (14), 115 (7), 87 (10), 78 (100), 70 (34), 69 (31), 66 (13), 65 (11), 41 (19).

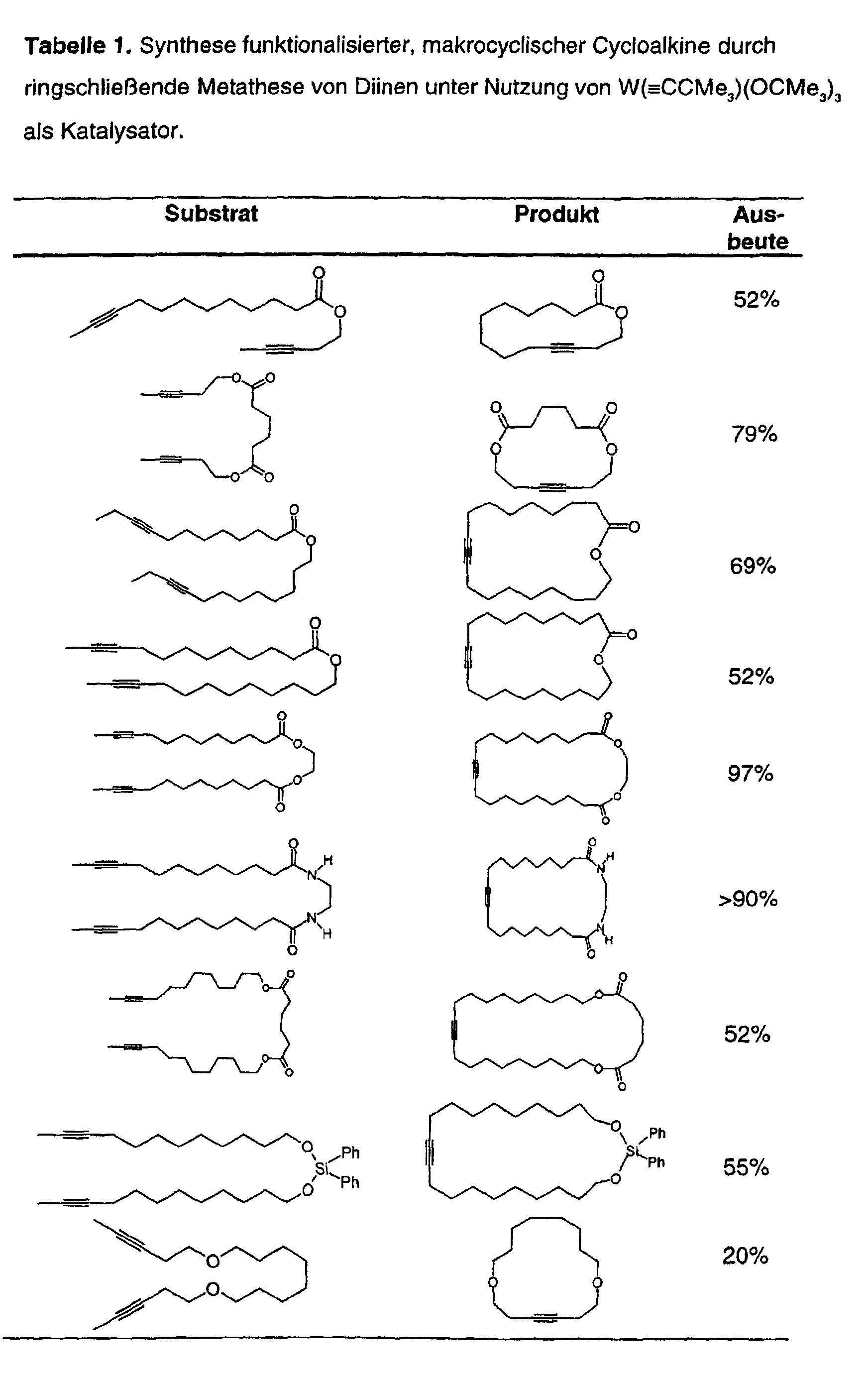

## Patentansprüche

1. Verfahren zur Darstellung von carbo- oder heterocyclischen Produkten mit 12 oder mehr Ringatomen durch ringschließende Metathesereaktionen, **dadurch gekennzeichnet, daß** Diin-Substrate in Gegenwart von einem oder mehreren homogen oder heterogen im Reaktionsmedium vorliegenden Alkinmetathese-Katatysatoren umgesetzt werden.

2. Verfahren nach Anspruch 1, wobei die Diin-Substrate eine oder mehrere funktionelle Gruppen In Form von Substituenten an der Kette oder Heteroatomen innerhalb der Kette enthalten; die genannten funktionellen Gruppen umfassen verzweigte oder unverzweigte Alkylreste, aromatische oder nicht aromatische carbocyclische Ringe, aromatische oder nicht aromatische Stickstoff-, Sauerstoff-, Schwefel- oder Phosphorhaltige heterocyclische Ringe, Carbonsäuren, Ester, Ether, Epoxide, Silylether, Thioether, Thioacetale, Disulfide, Alkohole, Anhydride, Imine, Silylether, Silylenolether, Ammoniumsalze, Amine, Amide, Nitrile, Perfluoralkyl-Gruppen, gem-Dialkytgruppen, Alkene, Halogene, Ketone, Ketale, Aldehyde, Acetale, Carbamate, Carbonate, Urethane, Harnstoffe, Sulfonate, Sulfohe, Sulfonamide, Sulfoxide, Phosphate, Phosphonate, Nitro-Gruppen, Organosilan-Einheiten oder Metallzentren.

3. Verfahren nach Anspruch 1-2, wobei der verwendete Alkin-Metathesekatalysator ein Übergangsmetall-Alkylidin Komplex ist.

4. Verfahren nach Anspruch3, wobei der Übergangsmetall-Alkylidinkomplex in situ im Reaktionsmedium gebildet wird.

5. Verfahren nach Anspruch3, wobei der als Katalysator verwendete Übergangsmetall-Alkylidin Komplex eine Verbindung vom Typ M(≡ CR¹)(OR²)₃ ist, mit
M=Mo,W
R¹ = C1-C20 Alkyl, Aryl, Alkenyl, Alkylthio, Dialkylamino
R² = C1-C20 Alkyl, Aryl, CMe₃, CH(CF₃)₂, CMe₂CF₃, CMe(CF₃)₂, C(CF₃)₃, C₆H₃Me₂, C₆H₃i-Pr₂, C₆H₃t-Bu₂.

6. Verfahren nach Anspruch 5 mit
M=W
R¹ = CMe₃, Ph
R² = CMe₃, CH(CF₃)₂, CMe₂CF₃, CMe(CF₃)₂, C(CF₃)₃, C₆H₃Me₂, C₆H₃i-Pr₂, C₆H₃t-Bu₂.

7. Verfahren nach Anspruch 3, wobei der als Katalysator verwendete Übergangsmetall-Alkylidin Komplex eine Verbindung vom Typ Re(≡ CR¹)(=NAr)(OR²)₂ ist, mit
R¹ = C1-C20 Alkyl, Aryl, Alkenyl
Ar = C6-C20 Aryl
R² = C1-C20 Alkyl, Aryl, CMe₃, CH(CF₃)₂, CMe₂CF₃, CMe(CF₃)₂, C(CF₃)₃, C₆H₃Me₂, C₆H₃i-Pr₂, C₆H₃t-Bu₂.

8. Verfahren nach Anspruch 7 mit
R¹ = CMe₃, Ph
Ar = C6-C20 Aryl
R² = CMe₃, CH(CF₃)₂, CMe₂CF₃, CMe(CF₃)₂, C(CF₃)₃, C₆H₃Me₂, C₆H₃i-Pr₂, C₆H₃t-Bu₂.

9. Verfahren nach Anspruch 1-3, wobei der verwendete Alkin-Metathesekatalysator ein Komplex mit Metall≡Metall Dreifachbindung ist.

10. Verfahren nach Anspruch 9', wobei der verwendete Alkin-Metathesekatalysator ein Komplex vom Typ (RO)₃M≡M(OR)₃ ist, mit
M = Mo, W
R = C1-C20 Alkyl, CMe₃, CH(CF₃)₂, CMe₂CF₃, CMe(CF₃)₂, C(CF₃)₃.

11. Verfahren nach Anspruch 10 mit
R = CMe₃, CH(CF₃)₂, CMe₂CF₃, CMe(CF₃)₂, C(CF₃)₃.

12. Verfahren nach Anspruch 1-3, wobei der Alkinmetathesekatalysator aus M[N(R¹)Ar]₃ und einer Halogenverbindung vom Typ R²₂EX₂ oder R³₃SiX gebildet wird, mit
M = Mo, W
R¹ = C1-C20 Alkyl, sec-Alkyl, t-Alkyl, Cycloalkyl
Ar = C6-C20 Aryl
R² = H, F, Cl, Br, I, C1-C20 Alkyl, Aryl
E = C, Si
R³ = C1-C20 Alkyl, Aryl
X = F, Cl, Br, I.

13. Verfahren nach Anspruch 12. mit
M = Mo
R¹ = t-Bu, i-Pr
Ar = C₆H₅, C₆H₄Me, C₆H₃Me₂, C₆H₃(iPr)₂, C₆H₃(t-Bu)₂, C₆H₂Me₃
R² = H, F, Cl, Br, I, C₆H₅
R³ = Me, t-Bu, Ph, i-Pr.

14. Verfahren nach Anspruch 1-13, wobei die Diin-Substrate durch ein oder mehrere strukturelle Elemente konformativ für den Ringschluß prä-organisiert sind; die genannten strukturellen Elemente umfassen chirale Zentren, Wasserstoffbrückenbindungen, supramolekulare Strukturen, rigide Rückgrade, Koordination an Metallzentren.

15. Verfahren nach Anspruch 143, wobei die Diin-Substrate konformativ flexibel sind.

16. Verfahren nach Anspruch 1-15, wobei die Diin-Substrate in trägergebundener Form eingesetzt werden.

17. Verfahren nach Anspruch 1-16, wobei durch Wahl der Konzentration der Diin-Substrate in Lösung die Bildung des makrocyclischen Cycloalkins begünstigt ist.

18. Verfahren nach Anspruch 1-17) wobei die Reaktion bei Drücken kleiner als Atmosphärendruck durchgeführt wird.

19. Verfahren nach Anspruch 1-17, wobei durch das Durchleiten eines inerten Gasstroms durch das Reaktionsmedium Nebenprodukte abgeführt werden.

20. Verfahren nach Anspruch 1-10, wobei durch Additiva die Aktiviät der verwendeten Katalysatoren erhöht wird.

21. Verfahren nach Anspruch 20, wobei als Additiva Phenole, geminal-Dihalogenalkane (gem-Dihalogenalkane) oder Halogensilane eingesetzt werden.

22. Verfahren nach Anspruch 21, wobei als Additiva Phenol, Trifluormethylphenol, Bis(trifluormethyl)phenol, Fluorphenol, Difluorphenol, Pentafluorphenol, Chlorphenol, Dichlorphenol, Pentachlorphenol, Dichlormethan, Dibrommethan, Difodmethan, Chloroform, Bromoform, lodoform, Tetrachlorkohlenstoff, Tetrabromkohlenstoff, Tetraiodkohlenstoff, α,α-Dichlortoluol, Trimethylchlorsilan, Dimethyldichlorsilan, Trimethylbromsilan, Dimethyl(t-butyl)chlorsilan, Dimethylphenylchlorsilan eingesetzt werden.

23. Verfahren nach Anspruch 1-22, wobei die Diin-Substrate cyclodimerisiert werden.

24. Verfahren zur Darstellung von carbo- oder heterocyclischen Cycloalkenen mit 12 oder mehr Ringatomen, **dadurch gekennzeichnet, daß** nach Anspruch 1-23 dargestellte Cycloalkine selektiv in Cycloalkene mit einheitlicher Konfiguration der Doppelbindung überführt werden.

25. Verfahren nach Anspruch 24 zur Herstellung von Cycloalkenen mit (Z)-konfigurierter Doppelbindung.

26. Verfahren zur Darstellung von Epothilon oder Epothilon-Analoga, **dadurch gekennzeichnet, daß** ein funktionalisiertes Cycloalkin nach Anspruch 2 hergestellt und im Anschluß nach bekannten Verfahren in Epothilon oder Epothilon-Analoga umgesetzt wird.

27. Verfahren nach Anspruch 1-26, wobei die carbo- oder heterocyclischen Produkte Antibiotika, Pharmaka für die Human- oder Veterinärmedizin, Agrochemikalien, Pheromone, Kronenether, Geruchsstoffe, Parfuminhaltsstoffe, oder Geschmacksstoffe sind.

## Claims

1. A process for the preparation of carbo- or heterocyclic products having 9 or more ring atoms by ring-closing metathesis reactions, **characterized in that** diyne substrates are reacted in the presence of one or several alkyne metathesis catalysts present homogeneously or heterogeneously in the reaction medium.

2. The process according to claim 1, wherein the diyne substrates contain one or several functional groups in the form of substituents on the chain or heteroatoms within the chain; said functional groups comprise branched or unbranched alkyl moieties, aromatic or non-aromatic carbocyclic rings, aromatic or non-aromatic nitrogen, oxygen, sulfur or phosphorus containing heterocyclic rings, carboxylic acids, esters, ethers, epoxides, silyl ethers, thioethers, thioacetals, disulfides, alcohols, anhydrides, imines, silyl ethers, silylenol ethers, ammonium salts, amines, amides, nitriles, perfluoroalkyl groups, gem-dialkyl groups, alkenes, halogens, ketones, ketals, aldehydes, acetals, carbamates, carbonates, urethanes, ureas, sulfonates, sulfones, sulfonamides, sulfoxides, phosphates, phosphonates, nitro groups, organosilane moieties, or metal centers.

3. The process according to claims 1 to 2, wherein the used alkyne metathesis catalyst is a transition metal alkylidyne complex.

4. The process according to claim 3, wherein the transition metal alkylidyne complex is formed in situ within the reaction medium.

5. The process according to claim 3, wherein the transition metal alkylidyne complex used as the catalyst is a compound of the type M(≡CR¹)(OR²)₃ with
M = Mo, W
R¹ = C₁-C₂₀ alkyl, aryl, alkenyl, alkylthio, dialkylamino
R² = C₁₋₂₀ alkyl, aryl, CMe₃, CH(CF₃)₂, CMe₂CF₃, CMe(CF₃)₂, C(CF₃)₃, C₆H₃Me₂, C₆H₃i-Pr₂, C₆H₃t-Bu₂.

6. The process according to claim 5 with
M = W
R¹ = CMe₃, Ph
R² = CMe₃, CH(CF₃)₂, CMe₂CF₃, CMe(CF₃)₂, C(CF₃)₃, C₆H₃Me₂, C₆H₃i-Pr₂, C₆H₃t-Bu₂.

7. The process according to claim 3, wherein the transition metal alkylidyne complex used as the catalyst is a compound of the type Re(≡CR¹)(=NAr)(OR²)₂ with
R¹ = C₁-C₂₀ alkyl, aryl, alkenyl
Ar = C₆-C₂₀ aryl
R² = C₁-C₂₀ alkyl, aryl, CMe₃, CH(CF₃)₂, CMe₂CF₃, CMe(CF₃)₂, C(CF₃)₃, C₆H₃Me₂, C₆H₃i-Pr₂, C₆H₃t-Bu₂.

8. The process according to claim 7 with
R¹ = CMe₃, Ph
Ar = C₆-C₂₀ aryl
R² = CMe₃, CH(CF₃)₂, CMe₂CF₃, CMe(CF₃)₂, C(CF₃)₃, C₆H₃Me₂, C₆H₃i-Pr₂, C₆H₃t-Bu₂.

9. The process according to claims 1 to 3, wherein the used alkyne metathesis catalyst is a complex having a metal≡metal triple bond.

10. The process according to claim 9, wherein the used alkyne metathesis catalyst is a complex of the type (RO)₃M≡M(OR)₃ with
M = Mo, W
R = C₁-C₂₀ alkyl, CMe₃, CH(CF₃)₂, CMe₂CF₃, CMe(CF₃)₂, C(CF₃)₃.

11. The process according to claim 10 with
R = CMe₃, CH(CF₃)₂, CMe₂CF₃, CMe(CF₃)₂, C(CF₃)₃.

12. The process according to claims 1 to 3, wherein the alkyne metathesis catalyst is formed from M[N(R¹)Ar]₃ and a halogen compound of the type R²₂EX₂ or R³₃SiX with
M = Mo, W
R¹ = C₁-C₂₀ alkyl, sec-alkyl, t-alkyl, cycloalkyl
Ar = C₆-C₂₀ aryl
R² = H, F, Cl, Br, I, C₁₋₂₀ alkyl, aryl
E = C, Si
R³ = C₁-C₂₀ alkyl, aryl
X = F, Cl, Br, I.

13. The process according to claim 12 with
M = Mo
R¹ = t-Bu, i-Pr
Ar = C₆H₅, C₆H₄Me, C₆H₃Me₂, C₆H₃(iPr)₂, C₆H₃(t-Bu)₂, C₆H₂Me₃
R² = H, F, Cl, Br, I, C₆H₅
R³ = Me, t-Bu, i-Pr.

14. The process according to claims 1 to 13, wherein the diyne substrates are conformationally pre-organized for the ring closure by one or several structural elements; said structural elements comprise chiral centers, hydrogen bonds, supramolecular structures, rigid backbones, a coordination to metal centers.

15. The process according to claims 1 to 13, wherein the diyne substrates are conformationally flexible.

16. The process according to claims 1 to 15, wherein the diyne substrates are used in a supported form.

17. The process according to claims 1 to 16, wherein the formation of the macrocyclic cycloalkyne is favored by selecting the concentration of the diyne substrates in solution.

18. The process according to claims 1 to 17, wherein the reaction is carried out at pressures below atmospheric pressure.

19. The process according to claims 1 to 17, wherein side products are eliminated by passing an inert gas flow through the reaction medium.

20. The process according to claims 1 to 10, wherein the activity of the used catalysts is increased by additives.

21. The process according to claim 20, wherein phenols, geminal dihalogenalkanes (gem-dihalogenalkanes) or halogensilanes are used as additives.

22. The process according to claim 21, wherein phenol, trifluoromethylphenol, bls(trifluoromethyl)phenol, fluorophenol, difluorophenol, pentafluorophenol, chlorophenol, dichlorophenol, pentachlorophenol, dichloromethane, dibromomethane, diiodomethane, chloroform, bromoform, iodoform, tetrachlorocarbon, tetrabromocarbon, tetraiodocarbon, α,α-dichlorotoluene, trimethylchlorosilane, dimethyldichlorosilane, trimethylbromosilane, dimethyl(t-butyl)chlorosilane, dimethylphenylchlorosilane are used as additives.

23. The process according to claims 1 to 22, wherein the diyne substrates are cyclodimerized.

24. A process for the preparation of carbo- and heterocyclic cycloalkenes having 12 or more ring atoms, **characterized in that** cycloalkynes prepared according to claims 1 to 23 are selectively converted into cycloalkenes having a uniform double bond configuration.

25. The process according to claim 24 for the preparation of cycloalkenes having a (Z)-configured double bond.

26. A process for the preparation of epothilone or epothilone analogues, **characterized in that** a functionalized cycloalkyne according to claim 2 is prepared and thereafter converted into epothilone or epothilone analogues according to known procedures.

27. The process according to claims 1 - 26, wherein the carbo- or heterocyclic products are antibiotics, pharmaceuticals for human or veterinary medicine, pheromones, crown ethers, odorous substances, perfume ingredients, or flavoring agents.

## Revendications

1. Procédé de préparation de produits carbocycliques ou hétérocycliques de 12 atomes dans leur cycle ou plus, par réactions de métathèse avec fermeture de cycle, **caractérisé en ce que** l'on fait réagir des substrats diine en présence d'un ou de plusieurs catalyseurs de métathèse alcyne, homogènes ou hétérogènes, présents dans le milieu de réaction.

2. Procédé selon la revendication 1, dans lequel les substrats diine contiennent un ou plusieurs groupes fonctionnels sous forme de substituants sur la chaîne ou d'hétéroatomes à l'intérieur de la chaîne, lesdits groupes fonctionnels comprenant des restes alkyle ramifiés ou non ramifiés, des noyaux carbocycliques aromatiques ou non aromatiques, des noyaux hétérocycliques aromatiques ou non aromatiques contenant des atomes d'azote, d'oxygène, de soufre ou de phosphore, des acides carboxyliques, des esters, des éthers, des époxydes, des silyléthers, des thioéthers, des thioacétals, des bisulfures, des alcools, des anhydrides, des imines, des silylènoléthers, des sels d'ammonium, des aminés, des amides, des nitriles, des groupes perfluoroalkyle, des groupes gem-dialkyle, des alcènes, des halogènes, des cétones, des cétals, des aldéhydes, des acétals, des carbamates, des carbonates, des uréthanes, des urées, des sulfonates, des sulfones, des sulfamides, des sulfoxydes, des phosphates, des phosphonates, des groupes nitro, des motifs organosilane ou des centres métalliques.

3. Procédé selon la revendication 1 ou 2, dans lequel le catalyseur de métathèse alcyne est un complexe métal de transition-alkylidine.

4. Procédé selon la revendication 3, dans lequel le complexe métal de transition-alkylidine est formé *in situ* dans le milieu de réaction.

5. Procédé selon la revendication 3, dans lequel le complexe métal de transition-alkylidine utilisé comme catalyseur est un composé de type M(≡ CR¹)(OR²)₃, où
M = Mo, W,
R¹ est un alkyle en C₁ à C₂₀, aryle, alcényle, alkylthio, dialkylamino,
R² est un alkyle en C₁ à C₂₀, aryle, CMe₃, CH(CF₃)₂, CMe₂CF₃, CMe(CF₃)₂, C(CF₃)₃, C₆H₃Me₂, C₆H₃i-Pr₂, C₆H₃t-Bu₂.

6. Procédé selon la revendication 5, où
M=W,
R¹ = CMe₃, Ph,
R² = CMe₃, CH(CF₃)₂, CMe₂CF₃, CMe(CF₃)₂, C(CF₃)₃, C₆H₃Me₂, C₆H₃i-Pr₂, C₆H₃t-Bu₂.

7. Procédé selon la revendication 3, dans lequel le complexe métal de transition-alkylidine utilisé comme catalyseur est un composé de type Re(≡ CR¹)(=NAr)(OR²)₂, où
R¹ est un alkyle en C₁ à C₂₀, aryle, alcényle,
Ar est un aryle en C₆ à C₂₀,
R² est un alkyle en C₁ à C₂₀, aryle, CMe₃, CH(CF₃)₂, CMe₂CF₃, CMe(CF₃)₂, C(CF₃)₃, C₆H₃Me₂, C₆H₃i-Pr₂, C₆H₃t-Bu₂.

8. Procédé selon la revendication 7, où
R¹ = CMe₃, Ph,
Ar est un aryle en C₆ à C₂₀,
R² = CMe₃, CH(CF₃)₂, CMe₂CF₃, CMe(CF₃)₂, C(CF₃)₃, C₆H₃Me₂, C₆H₃i-Pr₂, C₆H₃t-Bu₂.

9. Procédé selon l'une des revendications 1 à 3, dans lequel le catalyseur de métathèse alcyne utilisé est un complexe avec une triple liaison métal≡métal.

10. Procédé selon la revendication 9, dans lequel le catalyseur de métathèse alcyne utilisé est un complexe de type (RO)₃M≡M(OR)₃, où
M = Mo, W
R est un alkyle en C₁ à C₂₀, CMe₃, CH(CF₃)₂, CMe₂CF₃, CMe(CF₃)₂, C(CF₃)₃.

11. Procédé selon la revendication 10, où R = CMe₃, CH(CF₃)₂, CMe₂CF₃, CMe(CF₃)₂, C(CF₃)₃.

12. Procédé selon l'une des revendications 1 à 3, dans lequel le catalyseur de métathèse alcyne est formé à partir de M[N(R¹)Ar]₃ et d'un composé halogène de type R²₂EX₂ ou R³₃SiX, où
M=Mo,W,
R¹ est un alkyle en C₁ à C₂₀, un sec.-alkyle, un tert.-alkyle, un cycloalkyle,
Ar est un aryle en C₆ à C₂₀,
R² = H, F, Cl, Br, I, un alkyle en C₁ à C₂₀, aryle,
E = C, Si,
R³ est un alkyle en C₁ à C₂₀, aryle,
X = F, Cl, Br, I.

13. Procédé selon la revendication 12, où
M=Mo,
R¹ = tert.-Bu, i-Pr,
Ar = C₆H₅, C₆H₄Me, C₆H₃Me₂, C₆H₃(iPr)₂, C₆H₃(t-Bu)₂, C₆H₂Me₃,
R² = H, F, Cl, Br, I, C₆H₅,
R³ = Me, tert.-Bu, Ph, i-Pr.

14. Procédé selon l'une des revendications 1 à 13, dans lequel les substrats diine sont de conformation pré-organisée pour la fermeture du cycle par l'intermédiaire d'un ou de plusieurs éléments structurels, lesdits éléments structurels comprenant des centres chiraux, des liaisons par pont hydrogène, des structures supramoléculaires, des squelettes rigides polymères, des coordinations sur les centres métalliques.

15. Procédé selon l'une des revendications 1 à 13, dans lequel les substrats diine sont de conformation flexible.

16. Procédé selon l'une des revendications 1 à 15, dans lequel les substrats diine sont utilisés sous forme liée à un support.

17. Procédé selon l'une des revendications 1 à 16, dans lequel on favorise la formation de la cycloalkine macrocyclique par le choix de la concentration en substrats diine dans la solution.

18. Procédé selon l'une des revendications 1 à 17, dans lequel la réaction est mise en oeuvre à des pressions inférieures à la pression atmosphérique.

19. Procédé selon l'une des revendications 1 à 17, dans l'equel les sous-produits sont évacués par l'introduction d'un courant de gaz inerte à travers le milieu de réaction.

20. Procédé selon l'une des revendications 1 à 10, dans lequel l'activité des catalyseurs utilisés est augmentée par des additifs.

21. Procédé selon la revendication 20, dans lequel on utilise comme additifs des phénols, des geminal-dihalogénoalcanes (gemdihalogénoalcanes) ou des halogénosilanes.

22. Procédé selon la revendication 21, dans lequel on utilise comme additifs du phénol, du trifluorométhylphénol, du bis(trifluorométhyl)phénol, du fluorophénol, du difluorophénol, du pentafluorophénol, du chlorophénol, du dichlorophénol, du pentachlorophénol, du dichlorométhane, du dibromométhane, du diiodométhane, du chloroforme, du bromoforme, de l'iodoforme, du tétrachlorure de carbone, du tétrabromure de carbone, du tétraiodure de carbone, de l'a-a-dichlorotoluène, du triméthylchlorosilane, du diméthyldichlorosilane, du triméthylbromosilane, du diméthyl(tert.-butyl)chlorosilane, du diméthylphénylchlorosilane.

23. Procédé selon l'une des revendications 1 à 22, dans lequel les substrats diine sont cyclodimérisés.

24. Procédé de préparation de cycloalcènes carbocycliques ou hétérocycliques de 12 atomes dans leur cycle ou plus, **caractérisé en ce que** les cycloalkines préparées selon l'une des revendications 1 à 23 sont transformées de façon sélective en cycloalcènes avec une configuration unitaire de la double liaison.

25. Procédé selon la revendication 24 de préparation de cycloalcènes avec une double liaison de configuration Z.

26. Procédé de préparation d'épothilon ou d'analogues d'épothilon, **caractérisé en ce que** l'on prépare une cycloalkine fonctionnalisée selon la revendication 2 et **en ce qu'**on la transforme selon des procédés connus en épothilon ou en analogues d'épothilon.

27. Procédé selon l'une des revendications 1 à 26, dans lequel les produits carbocycliques ou hétérocycliques sont des antibiotiques, des produits pharmaceutiques destinés à la médecine humaine ou à la médecine vétérinaire, des produits phytosanitaires, des phéromones, des éthers-couronnes, des substances odoriférantes, des composants de parfums ou des substances aromatisantes.
